# EUROPEAN PATENT SPECIFICATION

(11) **EP 1 782 785 B1**
(45) Date of publication and mention of the grant of the patent: **17.12.2008**
(21) Application number: 05023122.4
(22) Date of filing: 24.10.2005
(51) Int. Cl.: A61G 15/00

(54) **Item of furniture for gynaecological treatment**
Möbelstück für gynäkologische Behandlungen
Meuble pour traitement gynécologique

(43) Date of publication of application: 09.05.2007
(73) Proprietor: VIFRAM S.r.l., 10100 Torino (IT)
(72) Inventor: Melis, Gianluigi, 10100 Torino (IT)
(74) Representative: Lotti, Giorgio

(56) References cited:
- EP-A- 1 287 783
- DE-A1- 3 015 644
- US-A- 4 741 506
- US-A1- 2002 163 624
- PATENT ABSTRACTS OF JAPAN vol. 1999, no. 05, 31 May 1999 (1999-05-31) & JP 11 042258 A (TABUCHI KAZUHISA), 16 February 1999 (1999-02-16)

## Description

The present invention relates to an item of furniture for gynaecological treatment.

In general, during routine gynaecological treatment, such as check-ups, diagnostic examinations, or surgical operations, the doctors use a series of diagnostic and/or surgical equipment and instruments, which must be carefully organised in the immediate vicinity of a gynaecological chair so that they may be accessed in the shortest possible time.

This series of diagnostic and/or surgical equipment and instruments is usually also accompanied by instruments for providing illumination, such as, for example scialytic lamps, or by instruments which are used for monitoring and checking, such as, for example, monitors, video cameras and photographic equipment.

It is obvious that the doctors must have the above-mentioned equipment and instruments within easy reach, and they may have to be engaged beforehand in the organisation of the examination surgery and/or operating theatre in order to arrange all the things that they need in the vicinity of the gynaecological chair according to a pre-established order, which is usually substantially similar each time.

DE-3015644 discloses an item of furniture for gynaecological treatment comprising a support frame and defining an area for the gynaecological treatment itself; wherein the item of furniture comprising a number of compartments which are obtained in the said frame and which are organised around said area in such a way that they may each house respective diagnostic or surgical means relating to the treatment.

The aim of the present invention is to produce an item of furniture for gynaecological treatment, which will permit a rapid, lasting and efficient organisation of an examination and/or operating room in a gynaecological surgery of hospital.

According to the present invention, will be produced an item of furniture for gynaecological treatment according to claim 1.

The present invention will now be described with reference to the attached drawings, which illustrate a non-limiting form of embodiment of the present invention, in which:
- FIGURE 1 is a perspective frontal view of a preferred form of embodiment of the item of furniture for gynaecological treatment produced according to the details of the present invention;
- FIGURE 2 is a rear elevation view of the item of furniture which is shown in FIGURE 1; and
- FIGURE 3 is a perspective view on an enlarged scale of a detail of the item of furniture which is shown in FIGURE 1.

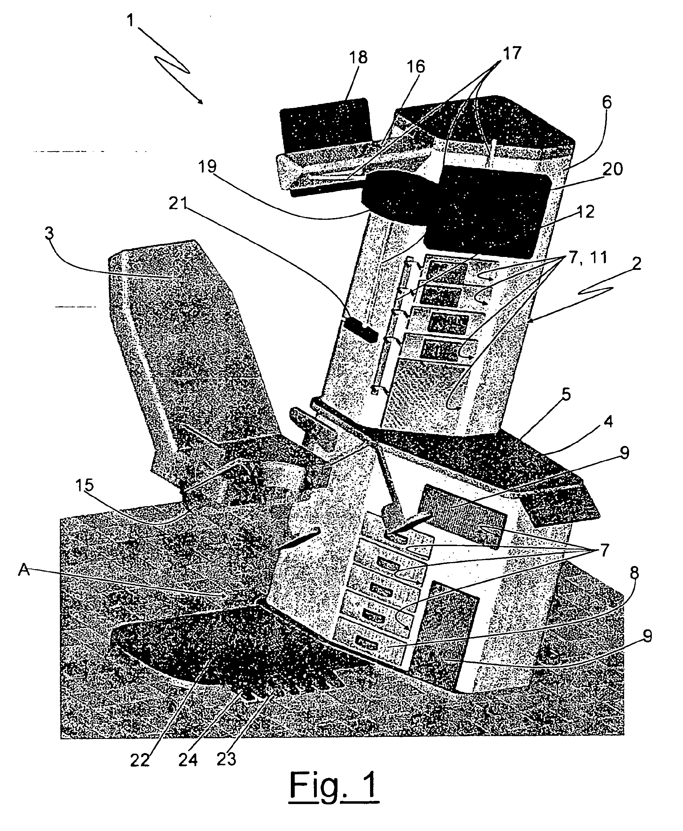

With reference to FIGURE 1, the number 1 indicates an item of furniture for gynaecological treatment in its entirety, or rather an item of furniture which may be used for all activities which might be inherent to gynaecological examinations or operations, and which is suitable for organising all the equipment and instruments which are necessary for such activity in an efficient manner.

The item of furniture 1 comprises a support frame 2 and defines an area A for the above-mentioned activity, inside which is arranged a gynaecological chair 3 which may in turn be integrated in the item of furniture 1 itself.

The frame 2 comprises a box-shaped base body 4 which is provided with an upper support shelf 5, and a bearing column 6, which is integral with the body 4, and which extends vertically upwards starting from the shelf 5.

In addition, the item of furniture 1 comprises a number of compartments 7, which are distributed between the body 4 and the column i6, and which are organised in the vicinity of the area A in order to each house a respective diagnostic and/or surgical and/or support instrument related to the above-mentioned gynaecological activity.

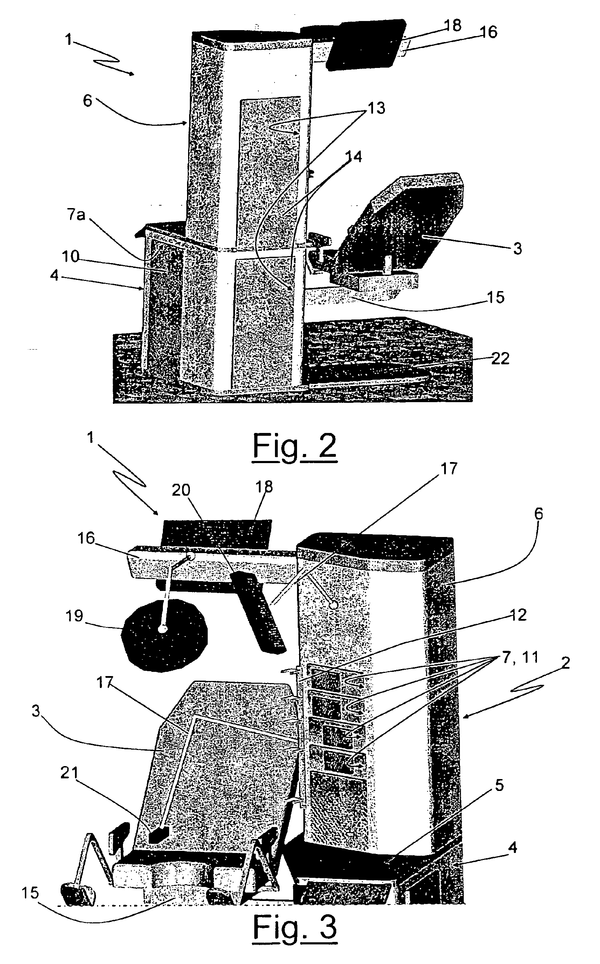

In particular, in the form of embodiment which is shown in the attached FIGURES by means of an example, the compartments 7 of the base body 4 comprise five drawers 8 which are arranged one on top of the other, and two further compartments 9 which are arranged laterally to the drawers 8 themselves, and which re suitable for housing, for example, a printer, and a personal computer, the keyboard of which may be arranged on the shelf 5. In addition, according to what is better illustrated in FIGURE 2, the body 4 comprises a rear compartment 7a, which is closed by a respective door 10, and is connected to the compartments 9 in order to permit the installation and/or maintenance of the above-mentioned personal computer.

The compartments 7 of the column 6 are instead defined by five compartments 11 for housing equipment such as, for example, a surgical aspirator, a colposcopy machine and the relative electronic control equipment, a halogen lamp for the emission of cold light, an ultrasound scan machine, and a radiofrequency electric bistoury machine. The compartments 11 are arranged one above the other along the column 6, they face the area A and the shelf 5, and are next to a support 12 for handpieces, which is an integral part of the item of furniture 1, and is suitable for supporting the equipment from the compartments 11 each time it is extracted, or having its own electronic control equipment arranged inside the compartments 11 themselves.

In addition, the column 6 presents, in the part which is opposite that which faces the area A, two further compartments 13, which are closed by respective doors 14, and which permit access to the compartments 11 for the checking and maintenance of the above-mentioned equipment.

According to what is better illustrated in FIGURE 3, the item of furniture 1 also comprises two support brackets 15 and 16 which are horizontal and parallel to each other, of which the bracket 15 is integral with the body 4, while the bracket 16 is integral with the column 6, and a number of extendible, rotating arms 17, which are anchored to the column 6 in order to each support relative equipment for monitoring, or for shooting, or for supporting additional diagnostic and/or surgical equipment.

In particular, the bracket 15 is arranged opposite the drawers 8 in relation to the compartments 9, and is anchored to the body 4 in order to extend towards the area A and to support the chair 3. Alternatively, the bracket 15 may be linked in recordable fashion to the body 4 in order to permit the regulation of the height of the chair 3 in the case that the chair 3 is not provided with such a registration.

The bracket 16 is anchored to the top of the column 6, and extends transverse from the column 6 itself both to support some of the arms 17, as well as to directly support a monitor 18 which is orientated in such a way as to favour the chair 3. In addition, the bracket 16 supports one of the arms 17, to the free end of which is connected a scialytic lamp 19.

A further two arms 17 extend directly from the column 6 in order to support a medical gynaecological monitor 20 which is shared by the equipment which is supported by the above-mentioned personal computer, and a colposcopy machine 21, which, as has been described above, may alternatively be housed in one of the compartments 9 and rested on the support 12.

Finally, the item of furniture 1 comprises a command footboard 22, which is connected to the body 4, and is arranged below the chair 3. The footboard 22 is provided with a number of activation pedals 23 which are connected to the above-mentioned equipment in order to activate it, and a number of activation and command pedals 24, which are connected to the chair 3 in order to control its respective operative position inside the area A.

According to what has been described above, it is obvious that the item of furniture 1 regroups in a single environment the substantial total of all the equipment which is most frequently used in a gynaecological surgery in such a way as to permit an optimal and efficient organisation of the work involved.

It is intended that the present invention should not be limited to the form of embodiment which is herein described and illustrated, which is to be considered as an example of a form of embodiment for an item of furniture for gynaecological treatment, and which may instead bye subject to further modifications relating to the shape and disposition of its parts, as well as to details pertaining to construction and assembly, and to the above-described equipment, which may be varied according to specific needs.

## Claims

1. Item of furniture (1) for gynaecological treatment comprising a support frame (2) and defining an area (A) for the gynaecological treatment itself; the item of furniture being **characterised by** the fact of comprising a number of compartments (7) which are obtained in the said frame (2) and which are organised around the said area (A) in such a way that they may each house respective diagnostic and/or surgical means and/or support means relating to the said gynaecological treatment;
a number of extendible rotating arms (17) are anchored to the said frame (2) in order that they may each support respective monitoring and/or shooting means, and/or additional said diagnostic and/or surgical means;
**characterised by** the fact of comprising a command footboard (22) which is arranged substantially at a level which is lower than that of the frame (2) and which is provided with a number of activation pedals (23) which are linked to the said monitoring means and/or shooting means, and/or diagnostic means, and/or surgical means.

2. Item of furniture according to Claim 1, **characterised by** the fact that the said footboard (22) also comprises a number of activation and command pedals (23) for the activation and command of a gynaecological chair (3) which is arranged inside the said area (A).

3. Item of furniture according to Claim 2, **characterised by** the fact of comprising a support bracket (15) which is connected to the said frame (2) in order to support the said gynaecological chair (3).

4. Item of furniture according to Claim 3, **characterised by** the fact of comprising a gynaecological chair (3) which is supported by the said support bracket (15), and which is connected to the said additional activation and command pedals (23) (24).

5. Item of furniture according to Claims 3 or 4, **characterised by** the fact that the said frame (2) comprises a box-shaped base (4) which is provided with a support shelf (5), and which presents a first part of the said compartments (7) some of which are provided with respective removable drawers.

6. Item of furniture according to Claim 5, **characterised by** the fact that the said frame (2) comprises a bearing column (6) which extends from the said box-shaped base (4) and from the said support shelf (5).

7. Item of furniture according to Claim 6, **characterised by** the fact that the said bearing column (6) presents a second part of the said compartments (7), and supports the said extendible rotating arms (17).

8. Item of furniture according to Claim 7, **characterised by** the fact of comprising a support arm (16) which is mounted substantially in correspondence with a top of the said bearing column (6) in order to support at least one monitor which faces the said area (A).

9. Item of furniture according to Claim 8, **characterised by** the fact that the said support arm (17) supports a scialytic lamp for which serves to illuminate the said area (A).

10. Item of furniture according to any of the Claims whatsoever from 7 to 9, **characterised by** the fact that the said bearing column (6) comprises a support (12) for handpieces which is arranged substantially adjacent to the said compartments (7).

## Patentansprüche

1. Möbelstück (1) für gynäkologische Behandlungen, das einen Halterahmen (2) umfasst und einen Bereich (A) für die gynäkologische Behandlung an sich definiert, wobei das Möbelstück **dadurch gekennzeichnet ist, dass** es eine Anzahl von Fächern (7) umfasst, die in dem Rahmen (2) erhalten werden und die um den Bereich (A) herum so organisiert sind, dass sie jeweils jeweilige diagnostische und/oder chirurgische Mittel aufnehmen und/oder Mittel, welche die gynäkologische Behandlung betreffen, halten,
eine Anzahl von ausziehbaren Dreharmen (17) an dem Rahmen (2) verankert ist, so dass sie jeweils jeweilige Überwachungs- und/oder Aufzeichnungsmittel und/oder zusätzliche diagnostische und/oder chirurgische Mittel halten können,
**dadurch gekennzeichnet, dass** ein Bedientrittbrett (22) inbegriffen ist, das im Wesentlichen auf einer Ebene angeordnet ist, die niedriger ist als die des Rahmens (2) und das mit einer Anzahl von Aktivierungspedalen (23) versehen ist, die mit den Überwachungsmitteln und/oder Aufzeichnungsmitteln und/oder diagnostischen Mitteln und/oder chirurgischen Mitteln verbunden sind.

2. Möbelstück nach Anspruch 1, **dadurch gekennzeichnet, dass** das Trittbrett (22) auch eine Anzahl von Aktivierungs- und Bedienpedalen (23) für die Aktivierung und Bedienung eines gynäkologischen Stuhls (3), der innerhalb des Bereichs (A) angeordnet ist, umfasst.

3. Möbelstück nach Anspruch 2, **dadurch gekennzeichnet, dass** es eine Haltestütze (15) umfasst, die mit dem Rahmen (2) verbunden ist, um den gynäkologischen Stuhl (3) zu halten.

4. Möbelstück nach Anspruch 3, **dadurch gekennzeichnet, dass** es einen gynäkologischen Stuhl (3) umfasst, der von der Haltestütze (15) gehalten wird und mit den zusätzlichen Aktivierungs- und Bedienpedalen (23) (24) verbunden ist.

5. Möbelstück nach Anspruch 3 oder 4, **dadurch gekennzeichnet, dass** der Rahmen (2) eine kastenförmige Basis (4) umfasst, die mit einer Halteplatte (5) versehen ist und einen ersten Teil der Fächer (7) aufweist, von denen einige mit jeweiligen entnehmbaren Schubladen versehen sind.

6. Möbelstück nach Anspruch 5, **dadurch gekennzeichnet, dass** der Rahmen (2) eine Tragsäule (6) umfasst, die sich von der kastenförmigen Basis (4) und von der Halteplatte (5) aus erstreckt.

7. Möbelstück nach Anspruch 6, **dadurch gekennzeichnet, dass** die Tragsäule (6) einen zweiten Teil der Fächer (7) aufweist und die ausziehbaren Dreharme (17) hält.

8. Möbelstück nach Anspruch 7, **dadurch gekennzeichnet, dass** es einen Haltearm (16) umfasst, der im Wesentlichen übereinstimmend mit einer Oberseite der Tragsäule (6) angebracht ist, um mindestens einen Monitor zu halten, der zu dem Bereich (A) hin weist.

9. Möbelstück nach Anspruch 8, **dadurch gekennzeichnet, dass** der Haltearm (17) eine OP-Leuchte hält, die dazu dient, den Bereich (A) auszuleuchten.

10. Möbelstück nach einem der Ansprüche 7 bis 9, **dadurch gekennzeichnet, dass** die Tragsäule (6) eine Halterung (12) für Handstücke umfasst, die im Wesentlichen benachbart zu den Fächern (7) angeordnet ist.

## Revendications

1. Meuble (1) pour traitement gynécologique comprenant un cadre de support (2) et définissant une zone (A) pour le traitement gynécologique lui-même; le meuble étant **caractérisé en ce qu'**il comprend un certain nombre de compartiments (7) qui sont obtenus dans ledit cadre (2) et qui sont organisés autour de ladite zone (A) de telle manière qu'ils puissent loger chacun des moyens de diagnostic et/ou chirurgicaux et/ou des moyens de support respectifs relatifs audit traitement gynécologique ;
un certain nombre de bras rotatifs extensibles (17) étant ancrés sur ledit cadre (2) afin de pouvoir supporter chacun des moyens de surveillance et/ ou de prise de vue; et/ou desdits moyens de diagnostic et/ou chirurgicaux supplémentaires respectifs ;
**caractérisé en ce qu'**il comprend une plate-forme de commande (22) qui est agencée sensiblement à un niveau qui est inférieur à celui du cadre (2) et qui est dotée d'un certain nombre de pédales d'activation (23) qui sont associées auxdits moyens de surveillance et/ou moyens de prise de vue, et/ou aux moyens de diagnostic et/ ou moyens chirurgicaux.

2. Meuble selon la revendication 1, **caractérisé en ce que** ladite plate-forme (22) comprend également un certain nombre de pédales d'activation et de commande (23) pour l'activation et la commande d'une chaise gynécologique (3) qui est agencée à l'intérieur de ladite zone (A).

3. Meuble selon la revendication 2, **caractérisé en ce qu'**il comprend un élément de support (15) qui est relié audit cadre (2) afin de supporter ladite chaise gynécologique (3).

4. Meuble selon la revendication 3, **caractérisé en ce qu'**il comprend une chaise gynécologique (3) qui est supportée par ledit élément de support (15), et qui est reliée aux dites pédales d'activation et de commande supplémentaires (23) (24).

5. Meuble selon les revendications 3 ou 4, **caractérisé en ce que** ledit cadre (2) comprend une base parallélépipédique (4) qui est dotée d'une étagère de support (5), et qui présente une première partie desdits compartiments (7) dont certains sont dotés de tiroirs amovibles respectifs.

6. Meuble selon la revendication 5, **caractérisé en ce que** ledit cadre (2) comprend une colonne de soutien (6) qui s'étend à partir de ladite base parallélépipédique (4) et de ladite étagère de support (5).

7. Meuble selon la revendication 6, **caractérisé en ce que** ladite colonne de soutien (6) présente une seconde partie desdits compartiments (7) et supporte lesdits bras rotatifs extensibles (17).

8. Meuble selon la revendication 7, **caractérisé en ce qu'**il comprend un bras de support (16) qui est monté sensiblement en correspondance avec une partie supérieure de ladite colonne de soutien (6) afin de supporter au moins un moniteur qui fait face à ladite zone (A).

9. Meuble selon la revendication 8, **caractérisé en ce que** ledit bras de support (17) supporte une lampe scialytique qui sert à éclairer ladite zone (A).

10. Meuble selon l'une quelconque des revendications 7 à 9, **caractérisé en ce que** ladite colonne de soutien (6) comprend un support (12) pour pièces à main qui est agencé de façon sensiblement adjacente aux dits compartiments (7).
